# EUROPEAN PATENT SPECIFICATION

(11) **EP 3 292 860 B1**
(45) Date of publication and mention of the grant of the patent: **17.04.2019**
(21) Application number: 16789340.3
(22) Date of filing: 06.05.2016
(51) Int. Cl.: A61K 9/107, A61K 31/337, A61K 47/14, A61P 35/00

(54) **CABAZITAXEL FAT EMULSION INJECTION, AND PREPARATION METHOD AND USE THEREOF**
CABAZITAXELFETTEMULSIONSINJEKTION UND HERSTELLUNGSVERFAHREN UND VERWENDUNG DAVON
INJECTION D'ÉMULSION GRASSE À BASE DE CABAZITAXEL, PROCÉDÉ DE PRÉPARATION ET UTILISATION ASSOCIÉS

(30) Priority: 06.05.2015 CN 201510227743
(43) Date of publication of application: 14.03.2018
(73) Proprietor: Jiangsu Tasly Diyi Pharmaceutical Co., Ltd., Huai'an, Jiangsu 223003 (CN)
(72) Inventor: CHEN, Jianming, Huai'an Jiangsu 223003 (CN); GAO, Bao'an, Huai'an Jiangsu 223003 (CN); ZHOU, Qinqin, Huai'an Jiangsu 223003 (CN); WANG, Guocheng, Huai'an Jiangsu 223003 (CN); YANG, Guojun, Huai'an Jiangsu 223003 (CN); LIU, Wenli, Huai'an Jiangsu 223003 (CN)
(74) Representative: Gulde & Partner
(86) International application number: PCT/CN2016/081245
(87) International publication number: WO 2016/177346

(56) References cited:
- CN-A- 101 019 832
- CN-A- 102 008 434
- CN-A- 103 006 558
- SHAO YANJIE ET AL: "Improving cabazitaxel chemical stability in parenteral lipid emulsions using cholesterol", EUROPEAN JOURNAL OF PHARMACEUTICAL SCIENCES, ELSEVIER, AMSTERDAM, NL, vol. 52, 20 October 2013 (2013-10-20), pages 1-11, XP028808107, ISSN: 0928-0987, DOI: 10.1016/J.EJPS.2013.09.024
- Anonymous: "JEVTANA (cabazitaxel) injection, for intravenous use Prescribing Information", , 30 September 2017 (2017-09-30), XP055435439, Retrieved from the Internet: URL:http://products.sanofi.us/jevtana/jevt ana.html [retrieved on 2017-12-15]
- ZHAO, MINGMING ET AL.: 'Research progress on drug-loaded in travenous lipid emulsions' JOURNAL OF SHENYANG PHARMACEUTICAL UNIVERSITY vol. 27, no. 12, 31 December 2010, pages 1014 - 1022, XP009502228

## Description

### FIELD OF THE INVENTION

The present invention relates to the technical field of medicine, and in particular to a cabazitaxel fat emulsion injection, and a preparation method and use thereof.

### BACKGROUND OF THE INVENTION

Cabazitaxel is a drug for treating prostate cancer developed by Sanofi-aventis, which is a chemical semi-synthetic taxoid small molecule compound, with the chemical name of 4-acetyloxy-2α-benzoyloxy-5β,20-epoxy-1-hydroxy-7β,10β-dimethoxy-9-oxotax-11-en-13α -yl(2R,3S)-3-t-butoxycarbonylamino-2-hydroxy-3-phenylpropionate and the structural formula as represented by the following Formula (I). This compound is white or off-white powder, almost insoluble in water and soluble in ethanol and other organic solvents.

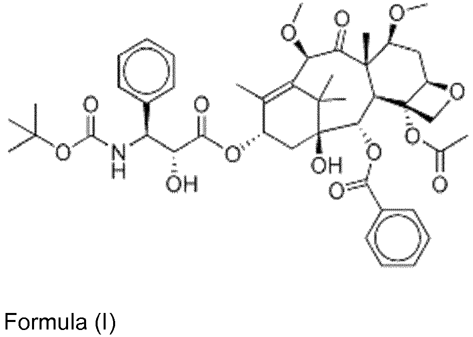

Cabazitaxel, as a microtubule inhibitor, can bind to tubulin, promote the assembly of microtubule dimers into microtubules, prevent the depolymerization process thereof, and inhibit the disassembly of microtubules, which leads to block cells in G₂ and M phases, thereby inhibiting the mitosis and proliferation of cancer cells. Meanwhile, cabazitaxel, as a novel second-generation taxane drug not only shows a broad spectrum of antitumor activity similar to the first-generation taxanes (such as Paclitaxel and Docetaxel), but also has the following unique advantages: (1) overcoming the resistance, and showing a significantly greater pharmacological activity in taxane-resistant cells; and (2) having an improved ability to cross the blood-brain barrier, showing a better pharmacological activity against central nervous system tumors (Patricia v rignaud, Dorothée Semiond, veronique Benning, et al., Preclinical profile of cabazitaxel [J]. Drug Des Devel Ther, 2014, 8:1851-1867) .

Currently, a cabazitaxel preparations for clinical application is a cabazitaxel injection solution, with the trade name of Jevtana and the strength of 60 mg/1.5 mL, comprising 60 mg of cabazitaxel and 1.5 mL of Tween-80, wherein the diluent is 5.7 mL of 13% (w/w) ethanol in water. This preparation was developed by Sanofi-Aventis and approved by the U.S. FDA for the treatment of prostate cancer on June 17, 2010.

However, this cabazitaxel injection solution has the following major disadvantages:
(1) Poor safety: The solvent composition is Tween-80. As reported in the references, intravenous injection of 0.3% or 0.35% Tween-80 can lead to grade 3 or more allergic reactions in Beagle dogs, such as fall of blood pressure, decreased heart rate, skin erythema, salivation and emesis, spasm and convulsion, and other abnormal reactions (Yongliang He, Yong Yi, Hongxing Wang, et al., Research on allergization in dogs caused by Traditional Chinese Medicine injection solution containing Tween-80 [J], Pharmacology and clinics of Chinese materia medica, 2005, 21(1):55-56; and Wenning Feng, Shunhan Xiao, Minghua Liu, et al., Research on toxicity of Chinese drug injection containing polysorbate 80 to different animals [J]. Journal of Luzhou Medical College, 2007, 30(2):92-94). For example, many adverse reactions occurred in clinical application of Docetaxel injection solution marketed (containing Tween-80), precisely because that Tween-80 is contained in the formulation, resulting in adverse reactions such as allergic reactions, myelosuppression, neurotoxicity, fluid retention, abnormal digestive system and the like, or even death caused by the adverse reactions (Xiaoqian Shi, Adverse reactions of Docetaxel and analysis thereon [J], China Medical Herald, 2009, 6(6):90-91). In clinical application, it is needed to administer corticosteroids, H2 antagonists, and antihistamine drugs in advance to patients as a precaution, while closely monitoring throughout the dosage regimen.
(2) Poor stability: The cabazitaxel injection solution has poor stability when being administrated in dilution, so it should be used within 30 minutes, which has a significant safety hazard.
(3) Inconvenient use: The use of this cabazitaxel injection solution requires rigorous training of medical professionals in terms of preparing, and it should simultaneously be filtered through a 0.22 µm filter membrane during infusion. Besides, infusion containers of polyvinyl chloride and polyurethane cannot be used, and real-time monitoring by a medical professional is also needed during infusion.

In view of the deficiencies of commercially available cabazitaxel injection solution in the aspects of safety, stability and the like in the prior art, it is necessary to improve the existing cabazitaxel injection solution, so that not only the water-solubility problem of cabazitaxel can be properly solved, but also the adverse effects caused by using Tween-80 can be avoided.

Lipid emulsion (also referred to as fat emulsion), as a carrier of insoluble drugs, has already attracted much attention. Currently, a large number of drug-loaded lipid emulsion injection solutions, such as Alprostadil injection, Propofol medium and long chain fat emulsion, Dexamethasone palmitate injection, Flurbiprofen Axetil injection and the like, have been widely used in clinical, and all of them show significant advantages in clinical application. Accordingly, fat emulsion has the unique advantages as a carrier of insoluble drugs.

Chinese Patent Application CN201210484494.6 disclosed a cabazitaxel lipid microsphere injection and a preparation method thereof. Wherein, in order to solve the solubility problem of cabazitaxel, cabazitaxel was firstly prepared into a phospholipid complex of cabazitaxel to increase the water-solubility and liposolubility, followed by preparing into a lipid microsphere injection in this reference. However, the biggest problem of this approach is that, a large number of toxic organic solvents such as chloroform, ethyl acetate, ethyl ether and the like need to be introduced during the preparation of the phospholipid complex of cabazitaxel, and it also needs to remove the organic solvents after the reaction is finished. The above processes are complicated and hard to control, and inevitably have the problem of residual organic solvents, which brings a safety hazard for clinical medication. Next, the oil for injection in this reference is selected from long chain triglycerides and medium chain triglycerides, and it specifically discloses soybean oil, safflower oil, sea buckthorn oil, *Oenothera biennis* oil, corn oil, *Brucea javanica* oil, coconut oil, perilla oil, grape seed oil, olive oil, castor oil, tea oil, cottonseed oil and palm oil, while not further performing the studies on adaptability. In fact, the majority of the oils for injection selected in this patent application belong to long chain oils while cabazitaxel has very low solubility or is even extremely insoluble in most of long chain oils. As a result, it is actually difficult to prepare a stable drug-loaded emulsion with high drug loading.

SHAO YANJIE et al., "Improving cabazitaxel chemical stability in parenteral lipid emulsions using cholesterol", Eur. J. Pharm. Sci., vol. 52(20), 2013, 1-11 discloses lipid emulsions comprising Cabazitaxel, lecithin, medium chain glycerides, poloxamer 188, glycerin and water. Although the preparation technologies of the drug-loaded fat emulsion are relatively mature, it still need to optimize the formulation and process which matches with it according to the inherent physicochemical properties of an active ingredient, to prepare a stable and safe drug-loaded fat emulsion.

### SUMMARY OF THE INVENTION

In view of the deficiencies in the existing cabazitaxel injection, in combination with the physicochemical properties of cabazitaxel, the applicant developed a cabazitaxel fat emulsion injection, which can not only properly solve the water-solubility problem of cabazitaxel, but also avoid the adverse effects caused by using Tween-80. Further, through painstaking research, the applicant found that cabazitaxel has the relatively higher solubility (about 50 mg/g) only in medium chain triglycerides, while has lower solubility in long chain oils (less than 10 mg/g), such as soybean oil, olive oil, castor oil, palm oil and the like. Therefore, based on the above solubility properties of cabazitaxel, the present invention particularly selected medium chain triglycerides as oils for injection in, thereby preparing a stable cabazitaxel fat emulsion injection with high drug loading.

Accordingly, the object of the present invention is to develop an intravenous injectable, safe and stable cabazitaxel fat emulsion injection.

As the first aspect of the present invention, the present invention provides a cabazitaxel fat emulsion injection, comprising cabazitaxel, medium chain triglyceride for injection and lecithin.

As the second aspect of the present invention, the present invention also provides a preparation method of the cabazitaxel fat emulsion injection comprising:
(1) weighing medium chain triglyceride for injection and a stabilizer according to the formulated amounts, mixed uniformly and heating to 50-80°C, followed by adding cabazitaxel thereto, and dissolve it with stirring or shearing at 50-80°C, to give an oil phase;
(2) dispersing a co-emulsifier and an isoosmotic adjusting agent (in the case that the target product is a cabazitaxel lyophilized emulsion, a lyophilized proppant is further included) into an appropriate amount of water for injection, heating to 50-80°C and dissolving them with stirring or shearing, followed by adding lecithin and dispersing it by shearing, to give a water phase;
(3) mixing the oil phase and the water phase at 50-80°C, and simultaneously emulsifying with a shear emulsifying machine for 5-15 minutes, to give a primary emulsion, which is subsequently made up with water for injection; and
(4) placing the primary emulsion into a high pressure homogenizer, further emulsifying at a homogenization pressure of 5000-20000 psi, preferably 7000-18000 psi, adjusting the pH to 3.0-7.0 with a pH adjusting agent, filtering, subpackaging, sealing and sterilizing, to give the cabazitaxel fat emulsion injection solution; or
(5) optionally, further preparing the cabazitaxel fat emulsion injection solution obtained into a cabazitaxel lyophilized emulsion by lyophilization.

As the third aspect of the present invention, the present invention further relates to the use of the cabazitaxel fat emulsion injection of the present invention in preparing a medicament for the treatment of prostate cancer.

As the fourth aspect of the present invention, the present invention also relates to the use of the cabazitaxel fat emulsion injection of the present invention in treating prostate cancer. As the fifth aspect of the present invention, the present invention further relates to a method for treating prostate cancer, comprising administering the cabazitaxel fat emulsion injection of the present invention to a patient in need thereof.

As the sixth aspect of the present invention, the present invention further relates to a cabazitaxel fat emulsion injection for treating prostate cancer, wherein the injection comprises cabazitaxel, medium chain triglyceride for injection and lecithin.

Specifically, the present invention includes any one fat emulsion composition for injection described in the claims.

In particular the cabazitaxel fat emulsion injection is characterised by being formulated by the following components, in weight/volume percentage (w/v):

| | |
|---|---|
| Cabazitaxel | 0.05-0.5% |
| Medium chain triglyceride for injection | 2-10% |
| Lecithin | 1-8% |
| Co-emulsifier | 0-0.5% |
| Isoosmotic adjusting agent | 0-2.2% |
| Stabilizer | 0-0.03% |
| Lyophilized proppant | 0-30% |
| pH adjusting agent | adjusting the pH to 3.0-7.0 |
| Water for injection | make up to 100%. |

wherein the stabilizer is oleic acid and/or sodium oleate and the lyophilized proppant is selected from one or more of lactose, sucrose, mannitol, Dextran 20, Dextran 40, Dextran 70, xylitol, sorbitol and trehalose.

The present invention is also directed to a preparation method of the cabazitaxel fat emulsion injection characterized in that the method comprises the following steps:
(1) weighing medium chain triglyceride for injection and a stabilizer according to the formulated amounts, mixed uniformly and heating to 50-80°C, followed by adding cabazitaxel thereto and dissolving it with stirring or shearing at 50-80°C, to give an oil phase;
(2) dispersing a co-emulsifier and an isoosmotic adjusting agent (in the case that the target product is a cabazitaxel lyophilized emulsion, a lyophilized proppant is further included) into an appropriate amount of water for injection, heating to 50-80°C and dissolving them with stirring or shearing, followed by adding lecithin and dispersing it by shearing, to give a water phase;
(3) mixing the oil phase and the water phase at 50-80°C, and simultaneously emulsifying with a shear emulsifying machine for 5-15 minutes, to give a primary emulsion, which is subsequently made up with water for injection; and
(4) placing the primary emulsion into a high pressure homogenizer, further emulsifying at a homogenization pressure of 5000-20000 psi, preferably 7000-18000 psi, adjusting the pH to 3.0-7.0 with a pH adjusting agent, filtering, subpackaging, sealing and sterilizing, to give the cabazitaxel fat emulsion injection solution; or
(5) optionally, further preparing the cabazitaxel fat emulsion injection solution obtained in step (4) into a cabazitaxel lyophilized emulsion by lyophilization.

Compared with commercially available cabazitaxel injections, the cabazitaxel fat emulsion injection provided in the present invention does not comprise any solubilizers having toxic and side effects such as Tween-80, and this is of great clinical significance. Compared with the prior art, the preparation method of the present invention omits the process of preparing into phospholipid complex, and avoids the step of adding a large number of toxic organic solvents such as chloroform, ethyl acetate, ethyl ether and the like required in the preparation of the cabazitaxel phospholipid complex, and the step of removing the organic solvents after the reaction is finished. These processes are complicated and hard to control, and inevitably have the problem of residual organic solvents. Therefore, the cabazitaxel fat emulsion injection of the present invention and the preparation method thereof can increase the drug safety of the product and the safety of the production process thereof, and also simplify the production process of the preparation. The cabazitaxel fat emulsion preparation prepared by the method of the present invention has the following advantages: simple preparation process, not using toxic and harmful organic solvents, and excellent stability and safety, which has a good prospect in social and economic benefits.

The inventor found that cabazitaxel has larger solubility and better stability in medium chain triglyceride for injection (please refer to Example 1). Thus, the solubility problem of cabazitaxel can be solved without the need of preparing into a phospholipid complex. In this way, the followings are avoided: (a) a large number of toxic organic solvents such as chloroform, ethyl acetate, ethyl ether and the like need to be introduced during the preparation of the cabazitaxel phospholipid complex; and (b) the organic solvents need to be removed after the reaction is finished, and the processes are complicated and hard to control, and inevitably have the problem of residual organic solvents. The production process of the preparation is also simplified.

### DETAILED EMBODIMENTS OF THE INVENTION

Hereinafter, the technical solutions of the present invention will be illustrated by specific Examples, but the protection scope of the present invention is not limited to the scope of the Examples. The reagents used are commercially available. In the Examples, unless specifically stated, "%" refers to weight/volume percentage concentration (w/v).

### Test Example 1: Investigation on the solubility of cabazitaxel in oil for injection

A fat emulsion for loading drugs was prepared, provided that the main drug thereof should has a certain degree of solubility in oils for injection, otherwise the stable fat emulsion for loading the drugs cannot be prepared. It is desirable that the cabazitaxel fat emulsion injection of the present invention is a long-term storable drug-loaded fat emulsion, which should at least be stable within 2 years. Thus, it is necessary to ensure that cabazitaxel has sufficient solubility in the oils for injection selected.

In view of the physicochemical properties of cabazitaxel, the present inventor investigated the solubility of cabazitaxel in several oils for injection (medium chain triglyceride, soybean oil, olive oil, castor oil and palm oil).

In this Test Example, appropriate amounts of cabazitaxel were weighed, respectively placed into medium chain triglyceride for injection, soybean oil, olive oil, castor oil and palm oil at 70°C, and heated with stirring, to observe the dissolved state thereof. The solutions with the drug dissolved were stored at a condition of 4-6°C for 72 hours, to perform a low temperature challenge test. The appearance changes of the solutions at 72 hours in the low temperature storage were observed. If the solutions appear cloudy, indicating that the fat emulsion prepared from oil solutions with this concentration has a risk of drug precipitation during the long-term storage. The results were shown in Table 1.

**Table 1 Solubility results of cabazitaxel in oils for injection**

| Oils for injection | Concentration (mg/g) | | | | | |
|---|---|---|---|---|---|---|
| | 10h | 20h | 30h | 40h | 50h | 60h |
| Medium chain triglyceride | Rapidly dissolved; the solution was still completely clear after refrigeration for 72h | Rapidly dissolved; the solution was still completely clear after refrigeration for 72h | Rapidly dissolved; the solution was still completely clear after refrigeration for 72h | Rapidly dissolved; the solution was still completely clear after refrigeration for 72h | Rapidly dissolved; the solution was still completely clear after refrigeration for 72h | Rapidly dissolved; the solution was slightly cloudy after refrigeration for 72h |
| Soybean oil | Still slightly cloudy after stirring for 1h | - | - | - | - | - |
| Olive oil | Still slightly cloudy after stirring for 1h | - | - | - | - | - |
| Castor oil | Still slightly cloudy after stirring for 1h | - | - | - | - | - |
| Palm oil | Still slightly cloudy after stirring for 1h | - | - | - | - | - |

| | | | | | | |
|---|---|---|---|---|---|---|
| "-": Completely insoluble | | | | | | |

As can be seen from the results of above Table 1, the solubility of cabazitaxel in the following oils for injection is very low: soybean oil, olive oil, castor oil and palm oil, and it cannot be fully dissolved at a concentration of 10 mg/g even with stirring at 70°C for 1h. However, its solubility is relatively larger in medium chain triglyceride for injection, and cabazitaxel even at a concentration of 60 mg/g can also be rapidly dissolved at 70°C. After storage at low temperature (4-6°C) for 72h, its solubility is about 50 mg/g.

### Example 1 Cabazitaxel lyophilized emulsion

50 g of medium chain triglyceride for injection and 0.3 g of oleic acid were weighed, mixed uniformly, heated to 70°C, and 1.5 g of cabazitaxel was added thereto and dissolved by stirring and shearing at 70°C, to give an oil phase; 150 g of lactose was weighed, dispersed into 700 mL of water for injection, heated to 70°C, and dissolved by stirring and shearing, and then 50 g of egg yolk lecithin was added therein and dispersed by shearing, to give a water phase; the oil phase and the water phase were mixed at 70°C and simultaneously emulsified with a shear emulsifying machine for 8 minutes, to give a primary emulsion, which was made up to 1000 mL with water for injection; and, the primary emulsion was placed into a high pressure homogenizer, further emulsified three times at a homogenization pressure of 15000 psi, adjusted the pH to 5.0 with hydrochloric acid, filtered and degermed respectively through 0.45 µm and 0.22 µm filter membranes, subpackaged, lyophilized and sealed, to give the cabazitaxel lyophilized emulsion.

### Example 2 Cabazitaxel lyophilized emulsion

50 g of medium chain triglyceride for injection and 0.3 g of oleic acid were weighed, mixed uniformly and heated to 70°C, and 1.5 g of cabazitaxel was added thereto and dissolved by stirring and shearing at 70°C, to give an oil phase; 2 g of Poloxamer 188 and 150 g of lactose were weighed, dispersed into 700 mL of water for injection, heated to 70°C, and dissolved by stirring and shearing, and then 50 g of egg yolk lecithin was added therein and dispersed by shearing, to give a water phase; the oil phase and the water phase were mixed at 70°C, and simultaneously emulsified with a shear emulsifying machine for 8 minutes, to give a primary emulsion, which was made up to 1000 mL with water for injection; and, the primary emulsion was placed into a high pressure homogenizer, further emulsified three times at a homogenization pressure of 10000 psi, adjusted the pH to 5.0 with hydrochloric acid, filtered and sterilized respectively through 0.45 µm and 0.22 µm filter membranes, subpackaged, lyophilized and sealed, to give the cabazitaxel lyophilized emulsion.

### Example 3 Cabazitaxel lyophilized emulsion

20 g of medium chain triglyceride for injection and 0.3 g of oleic acid were weighed, mixed uniformly and heated to 50°C, and 0.5 g of cabazitaxel was added thereto and dissolved by stirring and shearing at 50°C, to give an oil phase; 2 g of Poloxamer 188 and 100 g of lactose were weighed, dispersed into 800 mL of water for injection, heated to 50°C, and dissolved by stirring and shearing, and then 10 g of egg yolk lecithin was added thereto and dispersed by shearing, to give a water phase; the oil phase and the water phase were mixed at 50°C and simultaneously emulsified with a shear emulsifying machine for 15 minutes, to give a primary emulsion, which was made up to 1000 mL with water for injection; and, the primary emulsion was placed into a high pressure homogenizer, further emulsified six times at a homogenization pressure of 5000 psi, adjusted the pH to 4.0 with citric acid, filtered and degermed respectively through 0.8 µm and 0.22 µm filter membranes, subpackaged and lyophilized, to give the cabazitaxel lyophilized emulsion.

### Example 4 Cabazitaxel fat emulsion injection solution

100 g of medium chain triglyceride for injection was weighed and heated to 70°C, and 5 g of cabazitaxel was added thereto and dissolved by stirring and shearing at 70°C, to give an oil phase; 0.3 g of sodium oleate, 5 g of Poloxamer 188 and 22 g of glycerol were weighed, dispersed into 750 mL of water for injection, heated to 70°C, and dissolved by stirring and shearing, and then 80 g of egg yolk lecithin was added thereto and dispersed by shearing, to give a water phase; the oil phase and the water phase were mixed at 70°C and simultaneously emulsified with a shear emulsifying machine for 10 minutes, to give a primary emulsion, which was made up to 1000 mL with water for injection; and, the primary emulsion was placed into a high pressure homogenizer, further emulsified three times at a homogenization pressure of 16000 psi, adjusted the pH to 7.0 with disodium hydrogen phosphate and sodium dihydrogen phosphate, filtered respectively through 0.8 µm and 0.45 µm filter membranes, subpackaged, sealed, and sterilized for 30 minutes at 115°C, to give the cabazitaxel fat emulsion injection solution.

### Example 5 Cabazitaxel lyophilized emulsion

30 g of medium chain triglyceride for injection and 0.3 g of oleic acid were weighed, mixed uniformly and heated to 80°C, 10 g of soybean lecithin was added thereto and dissolved by stirring and shearing at 80°C, and then 1.0 g of cabazitaxel was added thereto and dissolved by stirring and shearing at 80°C, to give an oil phase; 5 g of Poloxamer 188, 100 g of lactose and 50 g of Dextran 40 were weighed, dispersed into 750 mL of water for injection, heated to 80°C, and dissolved by stirring and shearing, to give a water phase; the oil phase and the water phase were mixed at 80°C and simultaneously emulsified with a shear emulsifying machine for 5 minutes, to give a primary emulsion, which was made up to 1000 mL with water for injection; and, the primary emulsion was placed into a high pressure homogenizer, further emulsified three times at a homogenization pressure of 15000 psi, adjusted the pH to 6.0 with acetic acid and sodium acetate, filtered and degermed respectively through 1.2 µm, 0.45 µm and 0.22 µm filter membranes, subpackaged, lyophilized and sealed, to give the cabazitaxel lyophilized emulsion.

### Example 6 Cabazitaxel lyophilized emulsion

30 g of medium chain triglyceride for injection and 0.1 g of oleic acid were weighed, mixed uniformly and heated to 70°C, and 1.0 g of cabazitaxel was added thereto and dissolved by stirring and shearing at 70°C, to give an oil phase; 100 g of lactose and 50 g of sucrose were weighed, dispersed into 750 mL of water for injection, heated to 70°C and dissolved by stirring and shearing, and then 30 g of soybean lecithin was added and dispersed by shearing, to give a water phase; the oil phase and the water phase were mixed at 70°C, and simultaneously emulsified with a shear emulsifying machine for 5 minutes, to give a primary emulsion, which was made up to 1000 mL with water for injection; and, the primary emulsion was placed into a high pressure homogenizer, further emulsified five times at a homogenization pressure of 7000 psi, adjusted the pH to 4.0 with citric acid and sodium citrate, filtered and degermed respectively through 0.8 µm, 0.45 µm and 0.22 µm filter membranes, subpackaged, lyophilized and sealed, to give the cabazitaxel lyophilized emulsion.

### Example 7 Cabazitaxel lyophilized emulsion

60 g of medium chain triglyceride for injection and 0.3 g of oleic acid were weighed, mixed uniformly and heated to 60°C, and 1.5 g of cabazitaxel was added thereto and dissolved by stirring and shearing at 60°C, to give an oil phase; 100 g of lactose and 50 g of mannitol were weighed, dispersed into 700 mL of water for injection, heated to 60°C and dissolved by stirring and shearing, and then 60 g of egg yolk lecithin was added thereto and dispersed by shearing, to give a water phase; the oil phase and the water phase were mixed at 60°C, and simultaneously emulsified with a shear emulsifying machine for 10 minutes, to give a primary emulsion, which was made up to 1000 mL with water for injection; and, the primary emulsion was placed into a high pressure homogenizer, further emulsified twice at a homogenization pressure of 20000 psi, adjusted the pH to 3.0 with phosphoric acid, filtered and sterilized respectively through 0.45 µm and 0.22 µm filter membranes, subpackaged, lyophilized and sealed, to give the cabazitaxel lyophilized emulsion.

### Example 8 Cabazitaxel fat emulsion injection solution

50 g of medium chain triglyceride for injection was weighed and heated to 60°C, and 1.5 g of cabazitaxel was added thereto and dissolved by stirring and shearing at 60°C, to give an oil phase; 0.2 g of sodium oleate, 3 g of Poloxamer 188 and 22 g of glycerol were weighed, dispersed into 850 mL of water for injection, heated to 60°C, and dissolved by stirring and shearing, and then 40 g of egg yolk lecithin was added thereto and dispersed by shearing, to give a water phase; the oil phase and the water phase were mixed at 60°C, and simultaneously emulsified with a shear emulsifying machine for 8 minutes, to give a primary emulsion, which was made up to 1000 mL with water for injection; and, the primary emulsion was placed into a high pressure homogenizer, further emulsified four times at a homogenization pressure of 15000 psi, adjusted the pH to 3.0 with phosphoric acid, filtered respectively through 0.8 µm and 0.22 µm filter membranes, subpackaged, sealed, and sterilized for 15 minutes at 121°C, to give the cabazitaxel fat emulsion injection solution.

### Example 9 Cabazitaxel lyophilized emulsion

40 g of medium chain triglyceride for injection was weighed and heated to 70°C, and 1.5 g of cabazitaxel was added thereto and dissolved by stirring and shearing at 70°C, to give an oil phase; 130 g of lactose and 20 g of mannitol were weighed, dispersed into 700 mL of water for injection, heated to 70°C and dissolved by stirring and shearing, and then 20 g of egg yolk lecithin and 20 g of soybean lecithin were added thereto and dispersed by shearing, to give a water phase; the oil phase and the water phase were mixed at 70°C and simultaneously emulsified with a shear emulsifying machine for 7 minutes, to give a primary emulsion, which was made up to with 1000 mL with water for injection; and, the primary emulsion was placed into a high pressure homogenizer, further emulsified three times at a homogenization pressure of 15000 psi, adjusted the pH to 5.5 with phosphoric acid and sodium hydroxide, filtered and degermed respectively through 1.2 µm, 0.45 µm and 0.22 µm filter membranes, subpackaged, lyophilized and sealed, to give the cabazitaxel lyophilized emulsion.

### Example 10 Cabazitaxel lyophilized emulsion

50 g of medium chain triglyceride for injection was weighed and heated to 80°C, and 1.5 g of cabazitaxel was added thereto and dissolved by stirring and shearing at 80°C, to give an oil phase; 0.3 g of sodium oleate, 75 g of sorbitol and 75 g of xylitol were weighed, dispersed into 700 mL of water for injection, heated to 80°C and dissolved by stirring and shearing, and then 50 g of soybean lecithin was added thereto and dispersed by shearing, to give a water phase; the oil phase and the water phase were mixed at 80°C and simultaneously emulsified with a shear emulsifying machine for 5 minutes, to give a primary emulsion, which was made up to 1000 mL with water for injection; and, the primary emulsion was placed into a high pressure homogenizer, further emulsified three times at a homogenization pressure of 18000 psi, adjusted the pH to 5.0 with lactic acid, filtered and degermed respectively through 0.45 µm and 0.22 µm filter membranes, subpackaged, lyophilized and sealed, to give the cabazitaxel lyophilized emulsion.

### Example 11 Cabazitaxel lyophilized emulsion

50 g of medium chain triglyceride for injection was weighed and heated to 70°C, and 1.5 g of cabazitaxel was added thereto and dissolved by stirring and shearing at 70°C, to give an oil phase; 0.3 g of sodium oleate, 1 g of Poloxamer 188, 100 g of lactose, 30 g of Dextran 20 and 20 g of mannitol were weighed, dispersed into 700 mL of water for injection, heated to 70°C and dissolved by stirring and shearing, and then 20 g of egg yolk lecithin and 20 g of soybean lecithin were added thereto and dispersed by shearing, to give a water phase; the oil phase and the water phase were mixed at 70°C and simultaneously emulsified with a shear emulsifying machine for 8 minutes, to give a primary emulsion, which was made up to 1000 mL with water for injection; and, the primary emulsion was placed into a high pressure homogenizer, further emulsified three times at a homogenization pressure of 15000 psi, adjusted the pH to 6.0 with dipotassium hydrogen phosphate and potassium dihydrogen phosphate, filtered and degermed respectively through 1.2 µm, 0.45 µm and 0.22 µm filter membranes, subpackaged, lyophilized and sealed, to give the cabazitaxel lyophilized emulsion.

### Example 12 Cabazitaxel lyophilized emulsion

50 g of medium chain triglyceride for injection was weighed and heated to 60°C, and 1.5 g of cabazitaxel was added thereto and dissolved by stirring and shearing at 60°C, to give an oil phase; 120 g of lactose and 30 g of Dextran 70 were weighed, dispersed into 700 mL of water for injection, heated to 60°C and dissolved by stirring and shearing, and then 50 g of egg yolk lecithin was added and dispersed by shearing, to give a water phase; the oil phase and the water phase were mixed at 60°C and simultaneously emulsified with a shear emulsifying machine for 10 minutes, to give a primary emulsion, which was made up to 1000 mL with water for injection; and, the primary emulsion was placed into a high pressure homogenizer, further emulsified three times at a homogenization pressure of 16000 psi, adjusted the pH to 4.0 with hydrochloric acid, filtered and degermed respectively through 0.45 µm and 0.22 µm filter membranes, subpackaged, lyophilized and sealed, to give the cabazitaxel lyophilized emulsion.

### Example 13 Cabazitaxel fat emulsion injection solution

50 g of medium chain triglyceride for injection and 0.3 g of oleic acid were weighed, mixed uniformly and heated to 70°C, and 2.0 g of cabazitaxel was added thereto and dissolved by stirring and shearing at 70°C, to give an oil phase; 2.2 g of glycerol was weighed, dispersed into 800 mL of water for injection and heated to 70°C, and then 60 g of egg yolk lecithin was added thereto and dispersed by shearing, to give a water phase; the oil phase and the water phase were mixed at 70°C and simultaneously emulsified with a shear emulsifying machine for 6 minutes, to give a primary emulsion, which was made up to 1000 mL with water for injection; and, the primary emulsion was placed into a high pressure homogenizer, further emulsified three times at a homogenization pressure of 17000 psi, adjusted the pH to 5.0 with phosphoric acid and dipotassium hydrogen phosphate, filtered respectively through 0.8 µm and 0.22 µm filter membranes, subpackaged, sealed and sterilized for 45 minutes at 100°C, to give the cabazitaxel fat emulsion injection solution.

### Example 14 Cabazitaxel lyophilized emulsion

60 g of medium chain triglyceride for injection and 0.3 g of oleic acid were weighed, mixed uniformly and heated to 70°C, and 2.0 g of cabazitaxel was added thereto and dissolved by stirring and shearing at 70°C, to give an oil phase; 2 g of Poloxamer 188, 150 g of lactose and 30 g of mannitol were weighed, dispersed into 700 mL of water for injection, heated to 70°C and dissolved by stirring and shearing, and then 50 g of egg yolk lecithin was added thereto and dispersed by shearing, to give a water phase; the oil phase and the water phase were mixed at 70°C and simultaneously emulsified with a shear emulsifying machine for 5 minutes, to give a primary emulsion, which was made up to 1000 mL with water for injection; and, the primary emulsion was placed into a high pressure homogenizer, further emulsified three times at a homogenization pressure of 15000 psi, adjusted the pH to 6.0 with acetic acid and sodium acetate, filtered and degermed respectively through 0.8 µm and 0.22 µm filter membranes, subpackaged, lyophilized and sealed, to give the cabazitaxel lyophilized emulsion.

### Example 15 Cabazitaxel lyophilized emulsion

50 g of medium chain triglyceride for injection was weighed and heated to 70°C, and 2.0 g of cabazitaxel was added thereto and dissolved by stirring and shearing at 70°C, to give an oil phase; 0.3 g of sodium oleate and 150 g of lactose were weighed, dispersed into 700 mL of water for injection, heated to 70°C and dissolved by stirring and shearing, and then 50 g of soybean lecithin was added thereto and dispersed by shearing, to give a water phase; the oil phase and the water phase were mixed at 70°C and simultaneously emulsified with a shear emulsifying machine for 8 minutes, to give a primary emulsion, which was made up to 1000 mL with water for injection; and, the primary emulsion was placed into a high pressure homogenizer, further emulsified three times at a homogenization pressure of 16000 psi, adjusted the pH to 6.0 with citric acid and sodium hydroxide, filtered and degermed respectively through 0.45 µm and 0.22 µm filter membranes, subpackaged, lyophilized and sealed, to give the cabazitaxel lyophilized emulsion.

### Example 16 Cabazitaxel lyophilized emulsion

60 g of medium chain triglyceride for injection and 0.2 g of oleic acid were weighed, mixed uniformly and heated to 65°C, and 2.0 g of cabazitaxel was added thereto and dissolved by stirring and shearing at 65°C, to give an oil phase; 150 g of lactose and 20 g of sucrose were weighed, dispersed into 700 mL of water for injection, heated to 65°C and dissolved by stirring and shearing, and then 30 g of egg yolk lecithin and 30 g of soybean lecithin were added thereto and dispersed by shearing, to give a water phase; the oil phase and the water phase were mixed at 65°C and simultaneously emulsified with a shear emulsifying machine for 10 minutes, to give a primary emulsion, which was made up to 1000 mL with water for injection; and, the primary emulsion was placed into a high pressure homogenizer, further emulsified four times at a homogenization pressure of 16000 psi, adjusted the pH to 4.5 with lactic acid, filtered and degermed respectively through 1.2 µm, 0.45 µm and 0.22 µm filter membranes, subpackaged, lyophilized and sealed, to give the cabazitaxel lyophilized emulsion.

### Example 17 Cabazitaxel lyophilized emulsion

80 g of medium chain triglyceride for injection and 0.3 g of oleic acid were weighed, mixed uniformly and heated to 70°C, and 3.0 g of cabazitaxel was added thereto and dissolved by stirring and shearing at 70°C, to give an oil phase; 4 g of Poloxamer 188, 150 g of lactose, 20 g of sucrose and 30 g of mannitol were weighed, dispersed into 600 mL of water for injection, heated to 70°C and dissolved by stirring and shearing, and then 60 g of egg yolk lecithin was added and dispersed by shearing, to give a water phase; the oil phase and the water phase were mixed at 70°C and simultaneously emulsified with a shear emulsifying machine for 8 minutes, to give a primary emulsion, which was made up to 1000 mL with water for injection; and, the primary emulsion was placed into a high pressure homogenizer, further emulsified at a homogenization pressure of 15000 psi, adjusted the pH to 5.5 with acetic acid and sodium acetate, filtered and degermed respectively through 0.45 µm and 0.22 µm filter membranes, subpackaged, lyophilized and sealed, to give the cabazitaxel lyophilized emulsion.

### Example 18 Cabazitaxel lyophilized emulsion

60 g of medium chain triglyceride for injection was weighed and heated to 70°C, and 3.0 g of cabazitaxel was added thereto and dissolved by stirring and shearing at 70°C, to give an oil phase; 3 g of Poloxamer 188, 100 g of lactose, 40 g of sorbitol and 40 g of xylitol were weighed, dispersed into 650 mL of water for injection, heated to 70°C and dissolved by stirring and shearing, and then 60 g of egg yolk lecithin was added thereto and dispersed by shearing, to give a water phase; the oil phase and the water phase were mixed at 70°C and simultaneously emulsified with a shear emulsifying machine for 7 minutes, to give a primary emulsion, which was made up to 1000 mL with water for injection; and, the primary emulsion was placed into a high pressure homogenizer, further emulsified three times at a homogenization pressure of 17000 psi, adjusted the pH to 6.5 with disodium hydrogen phosphate and sodium dihydrogen phosphate, filtered and degermed respectively through 0.8 µm and 0.22 µm filter membranes, subpackaged, lyophilized and sealed, to give the cabazitaxel lyophilized emulsion.

### Example 19 Cabazitaxel fat emulsion injection solution

90 g of medium chain triglyceride for injection and 0.3 g of oleic acid were weighed, mixed uniformly and heated to 70°C, and 4.0 g of cabazitaxel was added thereto and dissolved by stirring and shearing at 70°C, to give an oil phase; 5 g of Poloxamer 188 and 22 g of glycerol were weighed, dispersed into 800 mL of water for injection, heated to 70°C and dissolved by stirring and shearing, and then 60 g of egg yolk lecithin was added thereto and dispersed by shearing, to give a water phase; the oil phase and the water phase were mixed at 70°C and simultaneously emulsified with a shear emulsifying machine for 8 minutes, to give a primary emulsion, which was made up to 1000 mL with water for injection; and, the primary emulsion was placed into a high pressure homogenizer, further emulsified four times at a homogenization pressure of 15000 psi, adjusted the pH to 5.0 with citric acid, filtered respectively through 0.8 µm and 0.22 µm filter membranes, subpackaged, sealed, and sterilized for 10 minutes at 121°C, to give the cabazitaxel fat emulsion injection solution.

### Example 20 Cabazitaxel lyophilized emulsion

80 g of medium chain triglyceride for injection and 0.3 g of oleic acid were weighed, mixed uniformly and heated to 70°C, and 4.0 g of cabazitaxel was added thereto and dissolved by stirring and shearing at 70°C, to give an oil phase; 4 g of Poloxamer 188, 200 g of lactose and 100 g of trehalose were weighed, dispersed into 800 mL of water for injection, heated to 70°C and dissolved by stirring and shearing, and then 70 g of egg yolk lecithin was added thereto and dispersed by shearing, to give a water phase; the oil phase and the water phase were mixed at 70°C and simultaneously emulsified with a shear emulsifying machine for 10 minutes, to give a primary emulsion, which was made up to 1000 mL with water for injection; and, the primary emulsion was placed into a high pressure homogenizer, further emulsified three times at a homogenization pressure of 16000 psi, adjusted the pH to 6.0 with citric acid and sodium hydroxide, filtered and degermed respectively through 0.88 µm and 0.22 µm filter membranes, subpackaged, lyophilized and sealed, to give the cabazitaxel lyophilized emulsion.

### Test Example 2: Stability tests of the cabazitaxel fat emulsion injections

The samples prepared in Examples 2, 3, 4, 6, 8 and 14 were respectively taken. They were placed at 25°C±2°C and a humidity of 60%±10% for 6 months, and sampled at 0 month, 1 month, 2 months, 3 months and 6 months, respectively. Wherein, the lyophilized emulsions were redissolved with water for injection to the concentration of the solutions before lyophilization. The cabazitaxel fat emulsion injection solutions and the redissolved solutions of the lyophilized emulsions were respectively taken, and the changes in the pH values, particle sizes, and labeled percentage contents were emphasically investigated. The results were respectively shown in Tables 2, 3 and 4.

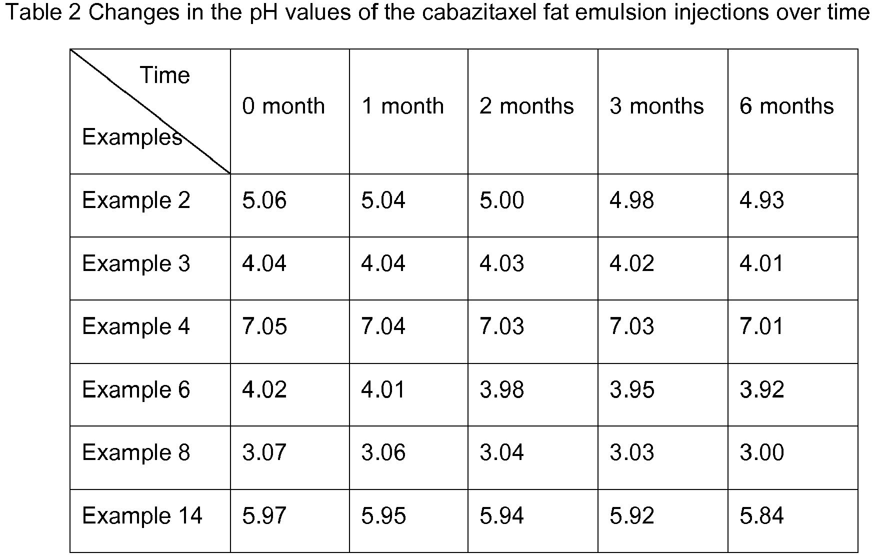

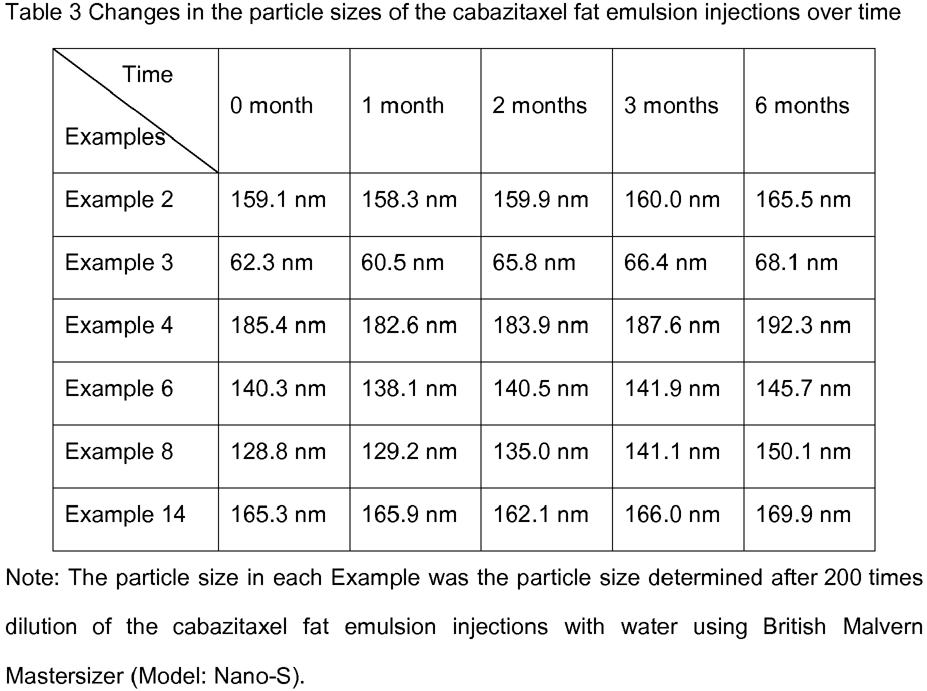

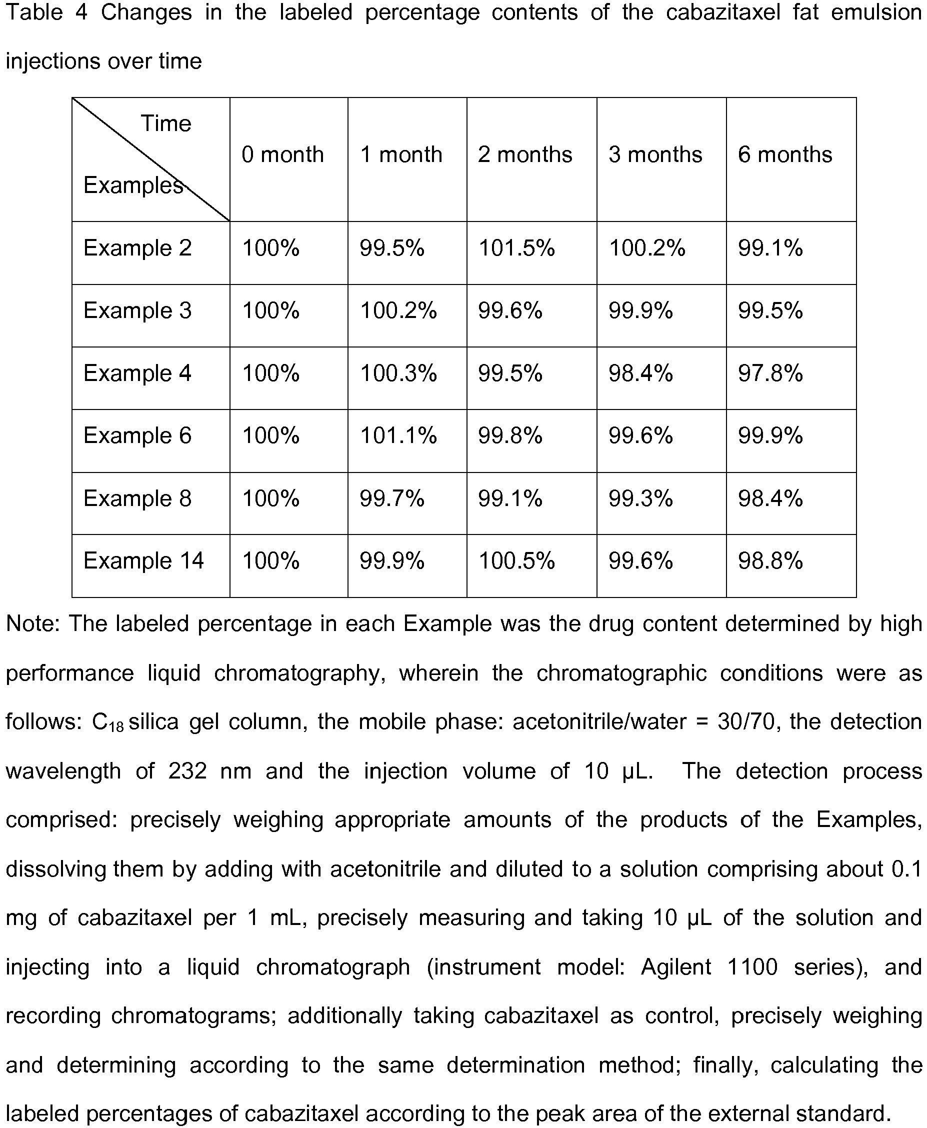

As can be seen from the above Tables 2-4, no obvious changes were observed in the pH values, particle sizes and labeled percentage contents of the cabazitaxel fat emulsion injections of the present invention when placement at 25°C±2°C and a humidity of 60%±10% for 6 months, indicating that the cabazitaxel fat emulsion injections of the present invention can be stored for longperiods of time and has stable quality. In particular, the cabazitaxel lyophilized fat emulsions after lyophilization (Examples 2, 3, 6 and 14) were more stable than the cabazitaxel fat emulsion injection solutions without lyophilization (Examples 4 and 8).

## Claims

1. A cabazitaxel fat emulsion injection consisting of the following components, in weight/volume percentage (w/v):
| | |
|---|---|
| Cabazitaxel | 0.05-0.5%, |
| Medium chain triglyceride for injection | 2-10%, |
| Lecithin | 1-8%, |
| Co-emulsifier | 0-0.5%, |
| Isoosmotic adjusting agent | 0-2.2%, |
| Stabilizer | 0-0.03%, |
| pH adjusting agent | adjusting the pH to 3.0-7.0, |
| a lyophilized proppant | 0-30%, |
| Water for injection | make up to 100%, |
wherein the stabilizer is oleic acid and/or sodium oleate; and
the lyophilized proppant is selected from one or more of lactose, sucrose, mannitol, Dextran 20, Dextran 40, Dextran 70, xylitol, sorbitol and trehalose.

2. The cabazitaxel fat emulsion injection according to claim 1, **characterized in that** the weight ratio of the cabazitaxel, the medium chain triglyceride for injection and the lecithin is (0.05-0.5):(2-10):(1-8), preferably (0.1-0.3):(3-8):(3-6).

3. The cabazitaxel fat emulsion injection according to claim 1, **characterized in that** the cabazitaxel fat emulsion injection solution comprises the following components, in weight/volume percentage (w/v):
| | |
|---|---|
| Cabazitaxel | 0.1-0.3%, |
| Medium chain triglyceride for injection | 3-8%, |
| Lecithin | 3-6%, |
| Co-emulsifier | 0.1-0.3%, |
| Isoosmotic adjusting agent | 0-2.2% |
| Stabilizer | 0.01-0.03%, |
| pH adjusting agent | adjusting the pH to 3.0-6.0, |
| Water for injection | make up to 100%. |

4. The cabazitaxel fat emulsion injection according to claim 1 or 2, **characterized in that** the cabazitaxel fat emulsion injection is a cabazitaxel lyophilized emulsion.

5. The cabazitaxel fat emulsion injection according to claim 4, **characterized in that** the cabazitaxel lyophilized emulsion is formulated by the following components, in weight/volume percentage (w/v):
| | |
|---|---|
| Cabazitaxel | 0.1-0.3%, |
| Medium chain triglyceride for injection | 3-8%, |
| Lecithin | 3-6%, |
| Co-emulsifier | 0.1-0.3%, |
| Isoosmotic adjusting agent | 0-2.2% |
| Stabilizer | 0.01-0.03%, |
| Lyophilized proppant | 0-20%, |
| pH adjusting agent | 3.0-6.0, |
| Water for injection | make up to 100%. |

6. The cabazitaxel fat emulsion injection according to any one of the preceding claims, **characterized in that** the lecithin is lecithin for injection, and selected from one or two of egg yolk lecithin and soybean lecithin;
the co-emulsifier is Poloxamer 188;
the isoosmotic adjusting agent is glycerol;
the pH adjusting agent is selected from one or more of citric acid, hydrochloric acid, acetic acid, phosphoric acid, lactic acid, sodium citrate, dipotassium hydrogen phosphate, disodium hydrogen phosphate, potassium dihydrogen phosphate, sodium dihydrogen phosphate, sodium acetate and sodium hydroxide;
the lyophilized proppant is lactose, sucrose and/or mannitol.

7. The cabazitaxel fat emulsion injection according to any one of the preceding claims, **characterized in that** the average particle size of the cabazitaxel fat emulsion injection is 60-250 nm, preferably 90-200 nm.

8. A preparation method of the cabazitaxel fat emulsion injection according to any one of the preceding claims, **characterized in that** the method comprises the following steps:
(1) weighing medium chain triglyceride for injection and a stabilizer according to the formulated amounts, mixed uniformly and heating to 50-80°C, followed by adding cabazitaxel thereto and dissolving it with stirring or shearing at 50-80°C, to give an oil phase;
(2) dispersing a co-emulsifier and an isoosmotic adjusting agent (in the case that the target product is a cabazitaxel lyophilized emulsion, a lyophilized proppant is further included) into an appropriate amount of water for injection, heating to 50-80°C and dissolving them with stirring or shearing, followed by adding lecithin and dispersing it by shearing, to give a water phase;
(3) mixing the oil phase and the water phase at 50-80°C, and simultaneously emulsifying with a shear emulsifying machine for 5-15 minutes, to give a primary emulsion, which is subsequently made up with water for injection; and
(4) placing the primary emulsion into a high pressure homogenizer, further emulsifying at a homogenization pressure of 5000-20000 psi, preferably 7000-18000 psi, adjusting the pH to 3.0-7.0 with a pH adjusting agent, filtering, subpackaging, sealing and sterilizing, to give the cabazitaxel fat emulsion injection solution; or
(5) optionally, further preparing the cabazitaxel fat emulsion injection solution obtained in step (4) into a cabazitaxel lyophilized emulsion by lyophilization.

9. A cabazitaxel fat emulsion injection according to any of claims 1-7 for use in treating prostate cancer.

## Patentansprüche

1. Cabazitaxel-Fettemulsionsinjektion, bestehend aus den folgenden Komponenten, in Gewicht/Volumen-Prozentanteil (Gew./Vol.):
| | |
|---|---|
| Cabazitaxel | 0,05 - 0,5 %, |
| Mittelkettiges Triglycerid zur Injektion | 2 - 10 %, |
| Lecithin | 1 - 8 %, |
| Co-Emulgator | 0 - 0,5 %, |
| Isoosmotisches Einstellmittel | 0 - 2,2 %, |
| Stabilisierungsmittel | 0 - 0,03 %, |
| pH-Einstellmittel | Einstellen des pH-Wertes auf 3,0 - 7,0, |
| ein lyophilisiertes Stützmittel | 0 - 30 %, |
| Wasser zur Injektion | Auffüllen auf 100 %, |
wobei das Stabilisierungsmittel Ölsäure und/oder Natriumoleat ist; und
das lyophilisierte Stützmittel ausgewählt ist aus einem oder mehreren von Laktose, Saccharose, Mannit, Dextran 20, Dextran 40, Dextran 70, Xylit, Sorbit und Trehalose.

2. Cabazitaxel-Fettemulsionsinjektion nach Anspruch 1, **dadurch gekennzeichnet, dass** das Gewichtsverhältnis von Cabazitaxel, dem mittelkettigen Triglycerid zur Injektion und dem Lecithin (0,05 - 0,5):(2 - 10):(1 - 8), vorzugsweise (0,1 - 0,3): (3 - 8): (3 - 6) beträgt.

3. Cabazitaxel-Fettemulsionsinjektion nach Anspruch 1, **dadurch gekennzeichnet, dass** die Cabazitaxel-Fettemulsionsinjektionslösung die folgenden Komponenten in Gewicht/Volumen-Prozentanteil (Gew./Vol.) umfasst:
| | |
|---|---|
| Cabazitaxel | 0,1 - 0,3 %, |
| Mittelkettiges Triglycerid zur Injektion | 3 - 8 %, |
| Lecithin | 3 - 6 %, |
| Co-Emulgator | 0,1 - 0,3 %, |
| Isoosmotisches Einstellmittel | 0 - 2,2 % |
| Stabilisierungsmittel | 0,01 - 0,03 %, |
| pH-Einstellmittel | Einstellen des pH- |
| | Wertes auf 3,0 - 6,0, |
| Wasser zur Injektion | Auffüllen auf 100 %. |

4. Cabazitaxel-Fettemulsionsinjektion nach Anspruch 1 oder 2, **dadurch gekennzeichnet, dass** die Cabazitaxel-Fettemulsionsinjektion eine lyophilisierte Cabazitaxel-Emulsion ist.

5. Cabazitaxel-Fettemulsionsinjektion nach Anspruch 4, **dadurch gekennzeichnet, dass** die lyophilisierte Cabazitaxel-Emulsion durch die folgenden Komponenten in Gewicht/Volumen-Prozentanteil (Gew./Vol.) formuliert ist:
| | |
|---|---|
| Cabazitaxel | 0,1 - 0,3 %, |
| Mittelkettiges Triglycerid zur Injektion | 3 - 8 %, |
| Lecithin | 3 - 6 %, |
| Co-Emulgator | 0,1 - 0,3 %, |
| Isoosmotisches Einstellmittel | 0 - 2,2 % |
| Stabilisierungsmittel | 0,01 - 0,03 %, |
| Lyophilisiertes Stützmittel | 0 - 20 %, |
| pH-Einstellmittel | 3,0 - 6,0, |
| Wasser zur Injektion | Auffüllen auf 100 %. |

6. Cabazitaxel-Fettemulsionsinjektion nach einem der vorhergehenden Ansprüche, **dadurch gekennzeichnet, dass** das Lecithin ein Lecithin zur Injektion ist und ausgewählt ist aus einem oder zwei von Eigelb-Lecithin und Soja-Lecithin;
der Co-Emulgator Poloxamer 188 ist;
das isoosmotische Einstellmittel Glycerin ist;
das pH-Einstellmittel ausgewählt ist aus einem oder mehreren von Zitronensäure, Salzsäure, Essigsäure, Phosphorsäure, Milchsäure, Natriumcitrat, Dikaliumhydrogenphosphat, Dinatriumhydrogenphosphat, Kaliumdihydrogenphosphat, Natriumdihydrogenphosphat, Natriumacetat und Natriumhydroxid;
das lyophilisierte Stützmittel Laktose, Saccharose und/oder Mannit ist.

7. Cabazitaxel-Fettemulsionsinjektion nach einem der vorhergehenden Ansprüche, **dadurch gekennzeichnet, dass** die durchschnittliche Partikelgröße der Cabazitaxel-Fettemulsionsinjektion 60 - 250 nm, vorzugsweise 90 - 200 nm beträgt.

8. Herstellungsverfahren der Cabazitaxel-Fettemulsionsinjektion nach einem der vorhergehenden Ansprüche, **dadurch gekennzeichnet, dass** das Verfahren die folgenden Schritte umfasst:
(1) Abwiegen von dem mittelkettigen Triglycerid zur Injektion und einem Stabilisierungsmittel gemäß den formulierten Mengen, gleichmäßig gemischt und erwärmt auf 50 - 80 °C, gefolgt von der Zugabe von Cabazitaxel dazu und dessen Auflösung unter Rühren oder Scheren bei 50 - 80 °C, um eine Ölphase zu erhalten;
(2) Dispergieren eines Co-Emulgators und eines isoosmotischen Einstellmittels (für den Fall, dass das Zielprodukt eine lyophilisierte Cabazitaxel-Emulsion ist, wird ferner ein lyophilisiertes Stützmittel eingeschlossen) in eine geeignete Menge Wasser zur Injektion, Erwärmen auf 50 - 80 °C und Auflösen unter Rühren oder Scheren, gefolgt durch Hinzufügen von Lecithin und Dispergieren von diesem durch Scheren, um eine Wasserphase zu erhalten;
(3) Mischen der Ölphase und der Wasserphase bei 50 - 80 °C und gleichzeitiges Emulgieren mit einer Scheremulgiermaschine für 5 - 15 Minuten, um eine primäre Emulsion zu erhalten, die anschließend mit Wasser zur Injektion aufgefüllt wird; und
(4) Einbringen der primären Emulsion in einen Hochdruckhomogenisator, weiteres Emulgieren bei einem Homogenisierungsdruck von 5.000 - 20.000 psi, vorzugsweise 7.000 - 18.000 psi, Einstellen des pH-Wertes auf 3,0 - 7,0 mit einem pH-Einstellmittel, Filtern, Unterverpacken, Versiegeln und Sterilisieren, um die Cabazitaxel-Fettemulsionsinjektionslösung zu erhalten; oder
(5) gegebenenfalls weiteres Präparieren der in Schritt (4) erhaltenen Cabazitaxel-Fettemulsionsinjektionslösung zu einer lyophilisierten Cabazitaxel-Emulsion durch Lyophilisierung.

9. Cabazitaxel-Fettemulsionsinjektion nach einem der Ansprüche 1 bis 7 zur Verwendung bei der Behandlung von Prostatakrebs.

## Revendications

1. Injection d'émulsion de graisse de cabazitaxel constituée des composants suivants, en pourcentage en poids/volume (p/v) :
| | |
|---|---|
| cabazitaxel | 0,05 - 0,5 %, |
| triglycéride à chaîne moyenne pour injection | 2 - 10 %, |
| lécithine | 1 - 8 %, |
| co-émulsifiant | 0 - 0,5 %, |
| agent d'ajustement isoosmotique | 0 - 2,2 %, |
| stabilisateur | 0 - 0,03 %, |
| agent d'ajustement de pH | ajustant le pH à 3,0 - 7,0 |
| agent de soutènement lyophilisé | 0 - 30 %, |
| eau pour injection | constituée à 100 %, |
où le stabilisateur est l'acide oléique et/ou l'oléate de sodium ; et
l'agent de soutènement lyophilisé est choisi parmi un ou plusieurs du lactose, du saccharose, du mannitol, du dextran 20, du dextran 40, du dextran 70, du xylitol, du sorbitol et du tréhalose.

2. Injection d'émulsion de graisse de cabazitaxel selon la revendication 1, **caractérisée en ce que** le rapport en poids du cabazitaxel, du triglycéride à chaîne moyenne pour injection et de la lécithine est de (0,05 - 0,5) : (2 - 10) : (1 - 8), de préférence de (0,1 - 0,3) : (3 - 8) : (3 - 6).

3. Injection d'émulsion de graisse de cabazitaxel selon la revendication 1, **caractérisée en ce que** la solution d'injection d'émulsion de graisse de cabazitaxel comprend les composants suivants, en pourcentage en poids/volume (p/v) :
| | |
|---|---|
| cabazitaxel | 0,1 - 0,3 %, |
| triglycéride à chaîne moyenne pour injection | 3 - 8 %, |
| lécithine | 3 - 6 %, |
| co-émulsifiant | 0,1 - 0,3 %, |
| agent d'ajustement isoosmotique | 0 - 2,2 %, |
| stabilisateur | 0,01 - 0,03 %, |
| agent d'ajustement de pH | ajustant le pH à 3,0 - 6,0 |
| eau pour injection | constituée à 100 %. |

4. Injection d'émulsion de graisse de cabazitaxel selon la revendication 1 ou 2, **caractérisée en ce que** l'injection d'émulsion de graisse de cabazitaxel est une émulsion lyophilisée de cabazitaxel.

5. Injection d'émulsion de graisse de cabazitaxel selon la revendication 4, **caractérisée en ce que** l'émulsion lyophilisée de cabazitaxel est formulée par les composants suivants, en pourcentage en poids/volume (p/v) :
| | |
|---|---|
| cabazitaxel | 0,1 - 0,3 %, |
| triglycéride à chaîne moyenne pour injection | 3 - 8 %, |
| lécithine | 3 - 6 %, |
| co-émulsifiant | 0,1 - 0,3 %, |
| agent d'ajustement isoosmotique | 0 - 2,2 %, |
| stabilisateur | 0,01 - 0,03 %, |
| agent de soutènement lyophilisé | 0 - 20 %, |
| agent d'ajustement de pH | 3,0 - 6,0, |
| eau pour injection | constituée à 100 %. |

6. Injection d'émulsion de graisse de cabazitaxel selon l'une quelconque des revendications précédentes, **caractérisée en ce que** la lécithine est une lécithine pour injection, et choisie parmi une ou deux de la lécithine de jaune d'oeuf et de la lécithine de soja ;
le co-émulsifiant est le poloxamère 188 ;
l'agent d'ajustement isoosmotique est le glycérol ;
l'agent d'ajustement de pH est choisi parmi un ou plusieurs de l'acide citrique, de l'acide chlorhydrique, de l'acide acétique, de l'acide phosphorique, de l'acide lactique, du citrate de sodium, de l'hydrogénophosphate dipotassique, de l'hydrogénophosphate disodique, du dihydrogénophosphate de potassium, du dihydrogénophosphate de sodium, de l'acétate de sodium et de l'hydroxyde de sodium ;
l'agent de soutènement lyophilisé est le lactose, le saccharose et/ou le mannitol.

7. Injection d'émulsion de graisse de cabazitaxel selon l'une quelconque des revendications précédentes, **caractérisée en ce que** la taille de particule moyenne de l'injection d'émulsion de graisse de cabazitaxel est de 60 à 250 nm, de préférence de 90 à 200 nm.

8. Procédé de préparation de l'injection d'émulsion de graisse de cabazitaxel selon l'une quelconque des revendications précédentes, **caractérisé en ce que** le procédé comprend les étapes suivantes :
(1) pesée du triglycéride à chaîne moyenne pour injection et d'un stabilisateur selon les quantités formulées, mélangés uniformément et chauffage à 50 à 80 °C, suivi par l'addition de cabazitaxel à ceux-ci et sa dissolution avec agitation ou cisaillement à 50 à 80 °C, pour donner une phase huileuse ;
(2) dispersion d'un co-émulsifiant et d'un agent d'ajustement isoosmotique (dans le cas où le produit cible est une émulsion lyophilisée de cabazitaxel, un agent de soutènement lyophilisé est en outre inclus) dans une quantité appropriée d'eau pour injection, chauffage à 50 à 80 °C et leur dissolution avec agitation ou cisaillement, suivi par l'addition de lécithine et sa dispersion par cisaillement, pour donner une phase aqueuse ;
(3) mélange de la phase huileuse et de la phase aqueuse à 50 à 80 °C, et émulsification simultanée avec une machine d'émulsification par cisaillement durant 5 à 15 minutes, pour donner une émulsion primaire, qui est ensuite constituée avec de l'eau pour injection ; et
(4) placement de l'émulsion primaire dans un homogénéiseur à haute pression, en émulsifiant en outre à une pression d'homogénéisation de 5000 à 20000 psi, de préférence de 7000 à 18000 psi, en ajustant le pH à 3,0 à 7,0 avec un agent d'ajustement de pH, en filtrant, en sous-conditionnant, en scellant et en stérilisant, pour donner la solution d'injection d'émulsion de graisse de cabazitaxel ; ou
(5) éventuellement, préparation en outre de la solution d'injection d'émulsion de graisse de cabazitaxel obtenue dans l'étape (4) en une émulsion lyophilisée de cabazitaxel par lyophilisation.

9. Injection d'émulsion de graisse de cabazitaxel selon l'une quelconque des revendications 1 à 7 destinée à être utilisée dans le traitement du cancer de la prostate.
